# EUROPÄISCHE PATENTANMELDUNG

(11) **EP 0 798 229 A1**
(43) Veröffentlichungstag der Anmeldung: **01.10.1997**
(21) Anmeldenummer: 97102830.3
(22) Anmeldetag: 21.02.1997
(51) Int. Cl.: B65D 65/38, A61F 13/15

(54) **Verpackungshülle**

(30) Priorität: 25.03.1996 DE 19611665
(71) Anmelder: 4P Folie Forchheim GmbH, D-91301 Forchheim (DE)
(72) Erfinder: Springer, Kurt, 91301 Forchheim (DE); Schmidt, Werner, 96129 Strullendorf (DE)
(74) Vertreter: Hutzelmann, Gerhard

(57) **Zusammenfassung**

Verpackungshülle(1) aus einer Kunststoffolie für eine Damenbinde(6), Slipeinlage od.dgl., welche mit einer selbstklebenden Fläche(7) versehen ist. Die Verpackungshülle(1) ist aus einer hochgeprägten Kunststoffolie(2) gebildet, die eine Dicke von etwa 30 µm aufweist. Die Gesamt-Stärke der Kunststoffolie einschließlich Prägung beträgt etwa 300 bis 400 µm.

## Beschreibung

Die Erfindung bezieht sich auf eine Verpackungshülle aus einer Kunststoffolie für eine Damenbinde, Slipeinlage od.dgl., welche mit einer selbstklebenden Fläche versehen ist.

Damenbinden, Slipeinlagen od.dgl. werden mit einer selbstklebenden Fläche versehen, um sie im Slip festlegen zu können. Diese selbstklebende Fläche muß bis zu ihrer Verwendung geschützt werden, da sie sonst ihre Klebkraft verliert. Dazu wird unmittelbar auf die selbstklebende Fläche ein Materialstreifen aufgebracht, der einseitig silikonisiert ist, um ihn wieder leicht ablösen zu können. Um diesen separaten Materialstreifen einzusparen, wurde auch bereits eine auf ihrer Innenseite silikonisierte Verpackungshülle eingesetzt.

Der Erfindung liegt die Aufgabe zugrunde, eine Verpackungshülle der genannten Art vorzuschlagen, mit der ein wirksamer Schutz der selbstklebenden Fläche erzielt wird, ohne daß eine vollständige Silikoniserung der gesamten Innenseite notwenig ist.

Diese Aufgabe wird erfindungsgemäß dadurch gelöst, daß die Verpackungshülle aus einer hochgeprägten Kunststoffolie gebildet ist.

Eine derart ausgetüstete Kunststoffolie klebt an der selbstklebenden Fläche und gibt ihr dabei einen ausreichenden Schutz; sie ist aber aufgrund ihrer Prägung wieder leicht abzulösen und beeinträchtigt dabei die Klebekraft nicht.

Bei einer vorteilhaften Ausgestaltung der Erfindung ist vorgesehen, daß die Folien-Dicke etwa 30 µm beträgt.

Damit ist nicht nur ein ausreichender Schutz der verpackten Damenbinde, Slipeinlage od.dgl. erreicht, sondern auch gewährleistet, daß die Verpackungshülle beim Ablösen von der selbstklebenden Fläche nicht zerreißt.

Eine vorteilhafte Ausgestaltung der Erfindung liegt auch darin, daß die Kunststoffolie der Verpackungshülle eine Gesamt-Stärke einschließlich Prägung von 300 bis 400 µm aufweist.

Mit dieser sehr hohen Prägung der Folie wird ein einwandfreies Ablösen der Verpackungshülle von der selbstklebenden Fläche erzielt.

Bei einer weiteren vorteilhaften Ausgestaltung der Erfindung ist vorgesehen, daß das Prägemuster pyramidenförmig ausgebildet ist.

Damit ist ein leichtes Ablösen der Verpackungshülle von der selbstklebenden Fläche gewährleistet.

Ebenfalls sehr vorteilhaft ist es, wenn gemäß einer weiteren Ausgestaltung der Erfindung das Prägemuster rautenförmig ausgebildet ist.

Auch mit einem derartigen Prägemuster ist ein leichtes Ablösen der Verpackungshülle von der selbstklebenden Fläche gewährleistet.

Als sehr vorteilhaft hat es sich auch erwiesen, wenn wenigstens eine der beiden Oberflächen der geprägten Folie verhältnismäßig glatt ausgebildet ist.

Eine glatte Oberfläche erleichtert das Ablösen von der selbstklebenden Fläche ganz wesentlich.

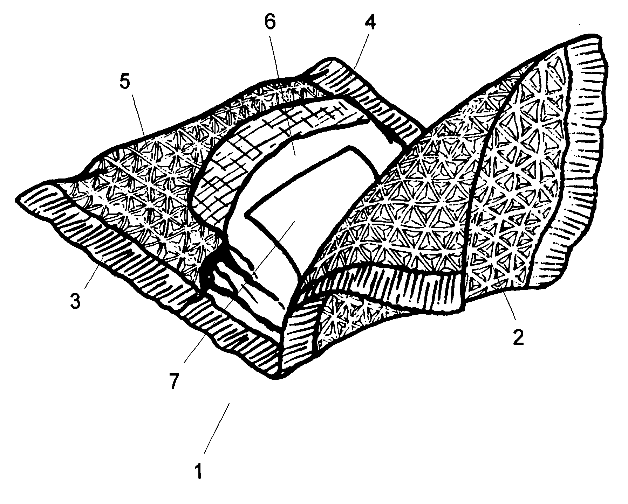

In der Zeichnung ist die Erfindung anhand eines Ausführungsbeispiels veranschaulicht. Dabei zeigt die Figur eine teilweise aufgetrennte Verpackungshülle mit einer darin verpackten Damenbinde.

Mit 1 ist eine Verpackungshülle bezeichnet, die aus einem Abschnitt 2 einer Kunststoffolie hergestellt ist und entlang von zwei Siegelnähten 3 und 4 seitlich verschlossen ist. Die beiden freien Enden sind großflächig übereinander geschlagen, wodurch ein ausreichend dichter Verschluß gegeben ist. Die Verpackungshülle ist ganzflächig mit einem Prägemuster 5 versehen, das im wesentlichen pyramidenförmig ausgebildet ist. Darüber hinaus ist die Verpackungshülle auf ihrer Innenseite mit einer verhältnismäßig glatten Oberfläche versehen.

In der Verpackungshülle 1 befindet sich eine Damenbinde 6, welche mit einem selbstklebenden Flächenabschnitt 7 versehen ist. Beim Verpacken der Damenbinde 6 legt sich die Verpackungshülle 1 an die selbstklebende Fläche 7 an und klebt dort fest, wodurch diese geschützt ist.

Beim Öffnen der Verpackungshülle kann die Kunststoffolie aufgrund ihres Prägemusters und ihrer glatten Oberfläche leicht von der selbstklebenden Fläche 7 abgelöst werden, ohne daß Folienteile darauf zurück bleiben.

Dies wird dadurch erreicht, daß die Folie aufgrund ihrer Gesamt-Stärke von 300 bis 400 µm und der daraus resultierenden großen Höhe des Prägemusters nur eine geringe Haftkraft auf der selbstklebenden Fläche 7 entwickelt. Die Folie ist dabei mit ihrer Dicke von 30 µm so stark, daß sie nicht einreißt und damit rückstandsfrei von der selbstklebenden Fläche abgelöst wird.

## Patentansprüche

1. Verpackungshülle(1) aus einer Kunststoffolie für eine Damenbinde(6), Slipeinlage od.dgl., welche mit einer selbstklebenden Fläche(7) versehen ist, **dadurch gekennzeichnet**, daß die Verpackungshülle(1) aus einer hochgeprägten Kunststoffolie(2) gebildet ist.

2. Verpackungshülle nach Anspruch 1, **dadurch gekennzeichnet**, daß die Folien-Dicke etwa 30 µm beträgt.

3. Verpackungshülle nach Anspruch 1 oder 2, **dadurch gekennzeichnet**, daß die Kunststoffolie(2) eine Gesamt-Stärke einschließlich Prägung von 300 bis 400 µm aufweist.

4. Verpackungshülle nach Anspruch 1, 2 oder 3, **dadurch gekennzeichnet**, daß das Prägemuster pyramidenförmig ausgebildet ist.

5. Verpackungshülle nach einem der Ansprüche 1 bis 3, **dadurch gekennzeichnet**, daß das Prägemuster rautenförmig ausgebildet ist.

6. Verpackungshülle nach einem der vorhergehenden Ansprüche, **dadurch gekennzeichnet**, daß wenigstens eine der beiden Oberflächen der geprägten Folie verhältnismäßig glatt ausgebildet ist.
